# EUROPÄISCHE PATENTSCHRIFT

(11) **EP 0 491 764 B1**
(45) Veröffentlichungstag und Bekanntmachung des Hinweises auf die Patenterteilung: **25.01.1995**
(21) Anmeldenummer: 90913388.6
(22) Anmeldetag: 06.09.1990
(51) Int. Cl.: C07C 69/14, C07C 69/54, C07C 69/78

(54) **BENZOPHENONETHER-ESTER, VERFAHREN ZU IHRER HERSTELLUNG, SOWIE IHRE VERWENDUNG ZUR VERBESSERUNG DER LICHTSTABILITÄT VON POLYESTERFÄRBUNGEN**
BENZOPHENONE ETHER ESTERS, PROCESS FOR MAKING THEM AND THEIR USE IN IMPROVING THE LIGHT-STABILITY OF POLYESTER DYES
ESTER D'ETHER DE BENZOPHENONE, SON PROCEDE DE PRODUCTION ET SON UTILISATION AFIN D'AMELIORER LA RESISTANCE A LA LUMIERE DE TEINTURES EN POLYESTER

(30) Priorität: 13.09.1989 DE 3930516
(43) Veröffentlichungstag der Anmeldung: 01.07.1992
(73) Patentinhaber: RIEDEL-DE HAEN AKTIENGESELLSCHAFT, D-30926 Seelze (DE)
(72) Erfinder: SCHULZ, Joachim, D-3255 Pohle (DE); BARTELS, Günter, D-3006 Burgwedel 1 (DE)
(74) Vertreter: Muley, Ralf, Dr.
(86) Internationale Anmeldenummer: EP9001493
(87) Internationale Veröffentlichungsnummer: WO9104243

(56) Entgegenhaltungen:
- EP-A- 0 182 056

## Beschreibung

Die vorliegende Erfindung betrifft Benzophenonether-ester, Verfahren zu ihrer Herstellung, ihre Verwendung zur Verbesserung der Lichtstabilität von Polyesterfärbungen sowie ein Färbeverfahren und Farbstoffpräparationen.

In der DE-AS 11 56 760 ist bereits ein Verfahren zur Verbesserung der Lichtechtheit von Polyesterfärbungen beschrieben, wobei Alkylether des 2,2',4,4'-Tetrahydroxybenzophenon zur Anwendung kommen. Dieses Verfahren weist allerdings eine Reihe von Nachteilen auf. So wird bei hellen Färbungen der Farbton und die Brillanz mehr oder weniger stark verschoben bzw. gemindert. Das Aufziehvermögen auf die Faser ist nicht erschöpfend genug, so daß Abwasserprobleme entstehen. Ferner neigen die dort beschriebenen Benzophenonderivate bei der üblichen Thermofixierung zur Migration.

Aus EP-A 254 987 und EP-A 309 909 sind auch bereits Verfahren zur Verbesserung der Lichtechtheit von Polyesterfärbungen unter Verwendung bestimmter Benzophenonether-ester bekannt.

Weiterhin sind aus EP 182 056 Benzophenonether-ester bekannt, die zum Schutz von Kunststoffen, wie beispielsweise Polyacrylsäureester, Polymethacrylsäureester, Polystyrol und ABS-Polymere, gegen die Einwirkung von UV-Licht verwendet werden.

Aufgabe vorliegender Erfindung ist es, Verbindungen zur Verfügung zu stellen, die eine Verbesserung der Lichtechtheit von Färbungen auf Polyestermaterialien bewirken, ohne daß das Aufziehvermögen des Farbstoffs sowie Farbtiefe und Brillanz der Färbung vermindert werden.

Diese Aufgabe wird überraschenderweise gelöst durch Verbindungen der allgemeinen Formel I 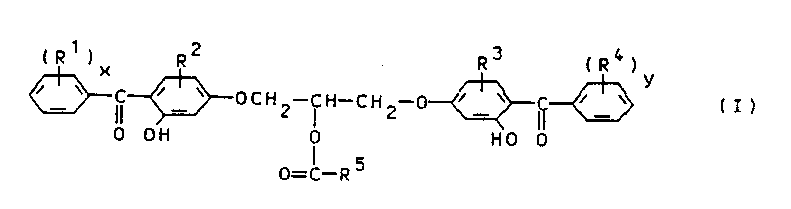
worin R¹ und R⁴ unabhängig voneinander (C₁-C₆)-Alkyl, Cyan, Fluor, Chlor, Brom, Trifluormethyl oder Wasserstoff;
R² und R³ unabhängig voneinander (C₁-C₆)-Alkyl oder Wasserstoff;
x und y unabhängig voneinander 0, 1 oder 2 bedeuten und
R⁵ für (C₁-C₁₂)-Alkyl; (C₃-C₈)-Cycloalkyl; durch (C₁-C₆)-Alkoxy, (C₂-C₇)-Alkoxycarbonyl, Amino, (C₁-C₆)-Alkylamino, Di-(C₁-C₆)-Alkylamino, Hydroxy, , Fluor, Chlor, Brom, Trifluormethyl oder Nitro substituiertes (C₁-C₁₂)-Alkyl oder (C₃-C₈)-Cycloalkyl; Phenyl oder durch (C₁-C₆)-Alkyl, (C₁-C₆)-Alkoxy, (C₂-C₇)-Alkoxycarbonyl, Amino, (C₁-C₆)-Alkylamino, Di-(C₁-C₆)-Alkylamino, Hydroxy, Cyan, Fluor, Chlor, Brom, Trifluormethyl oder Nitro substituiertes Phenyl steht.

Die genannten Alkyl-, Alkoxy-, Alkyloxycarbonyl-, Alkylamino- und Dialkylamino-Reste können geradkettig oder verzweigt sein.

Die Reste R¹ und R⁴ können in ortho-, meta- oder para-Stellung zur Carbonylgruppe stehen. Für x bzw. y = 2 können die Substituenten in 1,2-, 1,3- oder 1,4-Stellung zueinander stehen.

Die Reste R² und R³ können an alle drei freien Positionen des Sechsrings gebunden sein.

Bevorzugt sind Verbindungen der allgemeinen Formel I, in denen
R¹ und R⁴ unabhängig voneinander Wasserstoff, (C₁-C₃)-Alkyl, Cyan, Chlor oder Trifluormethyl,
R² und R³ unabhängig voneinander Wasserstoff oder (C₁-C₃)-Alkyl,
x und y unabhängig voneinander 0 oder 1 und
R⁵ (C₁-C₄)-Alkyl; Cyclopentyl, Cyclohexyl; durch (C₁-C₄)-Alkoxy, (C₂-C₅)-Alkoxycarbonyl, Amino, (C₁-C₄)-Alkylamino, Di-(C₁-C₄)Alkylamino, Hydroxy, , Fluor, Chlor, Brom, Trifluormethyl oder Nitro substituiertes (C₁-C₄)-Alkyl, Cyclopentyl oder Cyclohexyl; Phenyl oder durch (C₁-C₄)-Alkyl, (C₁-C₄)-Alkoxy, (C₂-C₅)-Alkoxycarbonyl, Amino, (C₁-C₄)-Alkylamino, Di-(C₁-C₄)-Alkylamino, Hydroxy, Cyan, Fluor, Chlor, Brom, Trifluormethyl oder Nitro substituiertes Phenyl bedeuten.

Besonders bevorzugt sind Verbindungen der allgemeinen Formel I, in denen
R¹, R², R³ und R⁴ Wasserstoff,
x und y O und
R⁵ Methyl oder Phenyl
bedeuten.

Die erfindungsgemäßen Verbindungen der allgemeinen Formel I können dadurch hergestellt werden, daß man eine Verbindung der allgemeinen Formel II 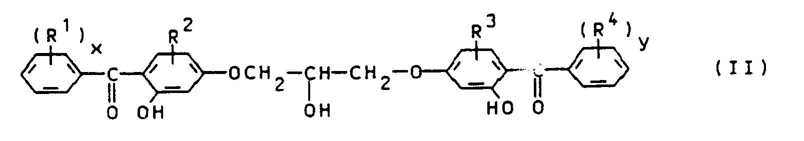
worin R¹, R², R³, R⁴, x und y wie oben definiert sind, mit einer Verbindung der allgemeinen Formel III 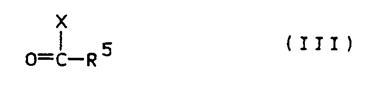
worin X für Hydroxy, Halogen, (C₁-C₄)-Alkoxy oder -OCOR⁵ steht und R⁵ wie oben definiert ist, umsetzt.

Die Umsetzung wird vorteilhafterweise bei Temperaturen von 0 bis 150^{o}C, besonders bevorzugt bei 50 bis 120^{o}C, durchgeführt.

Bevorzugt wird die Umsetzung in einem inerten organischen Lösungsmittel ausgeführt. Geeignete Lösungsmittel dieser Art sind insbesondere aromatische Kohlenwasserstoffe mit 6 bis 8 Kohlenstoffatomen, die mit einem Halogenatom, beispielsweise mit einem Chloratom, substituiert sein können, oder aliphatische Halogenkohlenwasserstoffe, beispielsweise Chlorkohlenwasserstoffe mit 1 bis 2 Kohlenstoffatomen. Besonders bevorzugte Lösungsmittel sind Benzol, Toluol, Xylol, Chlorbenzol, Chloroform, Tetrachlormethan und 1,2-Dichlorethan.

Das Lösungsmittel wird bevorzugt in Mengen von 1 bis 10 Liter, besonders bevorzugt 5 bis 8 Liter, pro Mol der Verbindung der allgemeinen Formel I verwendet.

Es ist besonders vorteilhaft, die Umsetzung in Gegenwart eines sauren Katalysators auszuführen, wobei insbesondere eine anorganische oder organische Säure in Betracht kommt. Bevorzugt sind organische Sulfonsäuren, wie beispielsweise p-Toluolsulfonsäure. Besonders bevorzugt sind organische Sulfonsäuren, die Halogenatome, insbesondere Fluoratome, aufweisen, wie beispielsweise Trifluormethansulfonsäure. Als saure Katalysatoren sind auch saure Ionenaustauscher geeignet.

Der Katalysator wird bevorzugt in einer Menge von 1 bis 10, besonders bevorzugt 3 bis 6, Gewichtsprozent, bezogen auf die Verbindung der allgemeinen Formel II eingesetzt.

Bevorzugt wird die Verbindung der allgemeinen Formel III in einem Überschuß bis zu 10 Mol pro Mol Verbindung der allgemeinen Formel II eingesetzt. Besonders bevorzugt ist ein Überschuß von 3 bis 8 Mol.

Bei den Verbindungen der allgemeinen Formel III handelt es sich um Carbonsäuren bzw. um Carbonsäurederivate, die käuflich und/oder nach dem Fachmann bekannten Methoden herstellbar sind. Beispiele für solche Carbonsäuren sind aliphatische Carbonsäuren wie Essigsäure, Propionsäure oder Buttersäure, und aromatische Carbonsäuren wie Benzoesäure.

Ebenso sind die Verbindungen der allgemeinen Formel I literaturbekannt (siehe z.B. CA 86(1977) 106170 t).

Die erfindungsgemäßen Verbindungen eignen sich in hervorragender Weise zur Verbesserung der Lichtechtheit von Polyesterfärbungen. Überraschenderweise wurde nun gefunden, daß sich auch die bereits aus der EP 182 056 bekannten und nach den dort beschriebenen Verfahren herstellbaren Verbindungen der allgemeinen Formel I, in denen R⁵ eine Gruppe der allgemeinen Formel A 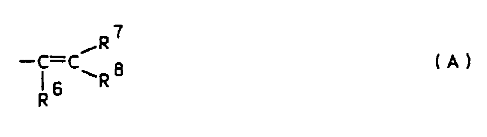
bedeutet, worin
R⁶ Wasserstoff, Phenyl, (C₁-C₁₂)-Alkyl oder Cyan und R⁷ und R⁸ unabhängig voneinander Wasserstoff, (C₁-C₈)-Alkyl, Phenyl oder durch (C₁-C₄)-Alkyl oder (C₁-C₄)-Alkoxy substituiertes Phenyl bedeuten oder gemeinsam einen Alkylenrest mit 4 oder 5 Kohlenstoffatomen bilden, zu diesem Zweck in hervorragender Weise eignen.

Die vorliegende Erfindung betrifft demnach auch die Verwendung einer Verbindung der allgemeinen Formel I 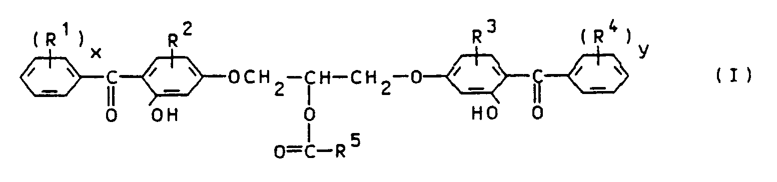
worin R¹ und R⁴ unabhängig voneinander (C₁-C₆)-Alkyl, Cyan, Fluor, Chlor, Brom, Trifluormethyl oder Wasserstoff;
R² und R³ unabhängig voneinander (C₁-C₆)-Alkyl oder Wasserstoff;
x und y unabhängig voneinander 0, 1 oder 2
bedeuten und
R⁵ für (C₁-C₁₂)-Alkyl; (C₃-C₈)-Cycloalkyl; durch (C₁-C₆)-Alkoxy, (C₂-C₇)-Alkoxycarbonyl, Amino, (C₁-C₆)-Alkylamino, Di-(C₁-C₆)-Alkylamino, Hydroxy, Cyan, Fluor, Chlor, Brom, Trifluormethyl oder Nitro substituiertes (C₁-C₁₂)-Alkyl oder (C₃-C₈)-Cycloalkyl; Phenyl oder durch (C₁-C₆)-Alkyl, (C₁-C₆)-Alkoy, (C₂-C₇)-Alkoxycarbonyl, Amino, (C₁-C₆)-Alkylamino, Di-(C₁-C₆)-Alkylamino, Hydroxy, Cyan, Fluor, Chlor, Brom, Trifluormethyl oder Nitro substituiertes Phenyl steht, oder eine Gruppe der allgemeinen Formel A 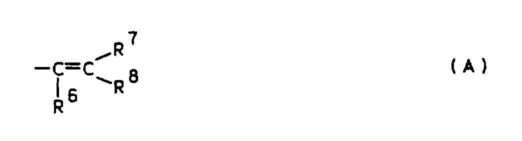
bedeutet, worin
R⁶ Wasserstoff, Phenyl, (C₁-C₁₂)-Alkyl oder Cyan und
R⁷ und R⁸ unabhängig voneinander Wasserstoff, (C₁-C₈)-Alkyl, Phenyl oder durch (C₁-C₄)-Alkyl oder (C₁-C₄)-Alkoxy substituiertes Phenyl bedeuten oder gemeinsam einen Alkylenrest mit 4 oder 5 Kohlenstoffatomen bilden, zur Verbesserung der Lichtechtheit von Polyesterfärbungen.

Es ist besonders vorteilhaft, wenn die Verbindungen der allgemeinen Formel I bereits beim Färben angewendet werden, das heißt im Färbebad enthalten sind. Die vorliegende Erfindung betrifft demnach auch ein Verfahren zum Färben von textilem Polyestermaterial mit Dispersionsfarbstoffen, dadurch gekennzeichnet, daß das Färbebad zur Verbesserung der Lichtechtheit der Färbung mindestens eine Verbindung der allgemeinen Formel I enthält, wobei sowohl die erfindungsgemäßen Verbindungen der allgemeinen Formel I als auch die aus der EP 182 056 bekannten Verbindungen der allgemeinen Formel I, worin R⁵ für die Gruppe A steht, eingeschlossen sind.

Unter textilem Polyestermaterial werden insbesondere Gebilde, wie beispielsweise Fasern, Fäden, Gewebe, Gewirke und Folien aus beispielsweise Polyethylenterephthalaten, Polybutylenterephthalaten oder Polyethylenglykolterephthalaten verstanden. Als Dispersionsfarbstoffe werden bevorzugt handelsübliche Dispersionsfarbstoffe, wie beispielsweise Azo-, Anthrochinon-, Methin-, Chinophthalon- oder Cumarinfarbstoffe, eingesetzt.

Es kann beispielsweise nach dem sogenannten Ausziehverfahren unter HT-Bedingungen, bei Kochtemperatur unter Carrier-Zusatz oder auch nach dem sogenannten Thermosolverfahren gefärbt werden. Die genannten Färbeverfahren sind dem Fachmann bekannt und in der einschlägigen Literatur beschrieben.

Die Färbebäder enthalten die Verbindungen der allgemeinen Formel I in Mengen von bevorzugt 0,1 bis 10, besondersbevorzugt 0,3 bis 5 Gewichtsprozent, bezogen auf das zu färbende Textilgut.

Die Verbindungen der allgemeinen Formel I können den Färbebädern beispielsweise als Pulver, sprühgetrocknetredispergierbar- oder als Flüssigzubereitung in Form einer Dispersion zugesetzt werden.

Besonders bevorzugt sind die Verbindungen der allgemeinen Formel I aber bereits in den Farbstoffpräparationen enthalten, aus denen die Färbebäder hergestellt werden.

Die vorliegende Erfindung betrifft demnach auch eine Farbstoffpräparation, dadurch gekennzeichnet, daß sie mindestens eine Verbindung der allgemeinen Formel I enthält, wobei sowohl die erfindungsgemäßen Verbindungen der allgemeinen Formel I als auch die aus der EP 182 056 bekannten Verbindungen der allgemeinen Formel I, worin R⁵ für die Gruppe A steht, eingeschlossen sind.

Die erfindungsgemäßen Farbstoffpräparationen sind flüssige oder pulverförmige Dispersionsfarbstoffpräparationen, die die Verbindungen der allgemeinen Formel I, bevorzugt in Mengen von 1 bis 50 Gewichtsprozent, besonders bevorzugt 1 bis 30 Gewichtsprozent, enthalten. Der Farbstoffgehalt beträgt bevorzugt 15 - 40 Gewichtsprozent, besonders bevorzugt 20 - 30 Gewichtsprozent.

Die Farbstoffpräparationen werden hergestellt, indem der Farbstoff in Gegenwart einer oder mehrerer Verbindungen der allgemeinen Formel I, eines oder mehrerer Dispergiermittel oder eines oder mehrerer Emulgatoren und gegebenenfalls in Gegenwart weiterer Hilfsmittel in geeigneten Mühlen gemeinsam gemahlen werden.

Geeignete Mühlen sind beispielsweise Kugel- oder Sandmühlen.

Der Mahlprozeß wird bei 0 bis 100^{o}C, bevorzugt bei 20 bis 70^{o}C, durchgeführt.

Soll eine pulverförmige Dispersion hergestellt werden, muß sich dem Mahlvorgang noch eine Sprühtrocknung anschließen.

Bei flüssigen Präparationen können die Verbindungen der allgemeinen Formel I unter der Voraussetzung, daß man sie gut unterrührt, auch nach dem Mahlen zugesetzt werden. Geeignete Dispergiermittel sind z. B. anionische oder nichtionische Dispergiermittel, die auch gemeinsam eingesetzt werden können. Anionische Dispergiermittel sind z. B. Kondensationsprodukte aus aromatischen Sulfonsäuren und Formaldehyd, insbesondere Kondensationsprodukte aus Alkylnaphthalinsulfonsäuren und Formaldehyd, Kondensationsprodukte aus gegebenenfalls substituiertem Phenol, Naphthalin- bzw. Naphtholsulfonsäuren, Formaldehyd und Natriumbisulfit, Alkalimetallsalze von Kondensationsprodukten, Formaldehyd und Harnstoff sowie Alkalimetallsalze von Ligninsulfonsäuren. Alkyl- oder Alkylarylsulfonate sowie Alkyl-aryl-polyglykol-ether-sulfate und insbesondere neutralisierte Säureester eines oxethylierten Novolacks. Nichtionische Dispergiermittel oder Emulgatoren sind z.B. Ethylen-oder Propylenoxid mit alkylierbaren Verbindungen, wie z.B. Fettalkoholen, Fettaminen, Fettsäuren, Phenolen, Alkylphenolen, Arylalkylphenolen, Arylalkylarylphenolen und Carbonsäure-amiden, wie z.B. Anlagerungsprodukte von 5 bis 10 Ethylenoxid-Einheiten an C₈-C₁₀-Alkylphenole.

Die genannten Dispergiermittel sind in flüssigen Farbstoffpräparationen zu 15 - 40 Gewichtsprozent, bevorzugt 20 - 30 Gewichtsprozent, enthalten, in pulverförmigen Farbstoffpräparationen zu 20 - 45 Gew.%.

Die erfindungsgemäßen Farbstoffpräparationen können auch noch weitere Hilfsmittel enthalten, z.B. solche, die als Oxidationsmittel wirken, wie z.B. Natrium-m-nitrobenzolsulfonat oder fungizide Mittel, wie z.B. Natrium-o-phenolphenolat und Natrium-penta-chlorphenolat. Als Pulver formulierte Farbstoffmischungen enthalten darüber hinaus noch andere Hilfsmittel wie z.B. Netz- oder Entstaubungsmittel. Die Farbstoffpräparationen enthalten die genannten Hilfsmittel in Mengen von 0 - 5 Gewichtsprozent, bevorzugt 0 - 2 Gewichtsprozent.

Färbungen, die nach dem erfindungsgemäßen Färbeverfahren, das heißt in Gegenwart der Verbindungen der allgemeinen Formel I, gefärbt werden, unterscheiden sich von solchen, die ohne Zusatz der Verbindungen der allgemeinen Formel I gefärbt wurden, im Farbton nicht oder nur unwesentlich. Sie zeichnen sich aber durch eine wesentlich höhere Lichtechtheit, insbesondere auch Heißlichtechtheit, aus. Somit werden auch die hohen Anforderungen, die an Färbungen im Automobilsektor (Sitzbezüge, Hutablagen usw.) gestellt werden, erfüllt und sogar übertroffen.

### Beispiel 1

### (1,3-Bis(4-benzoyl-3-hydroxyphenoxy)-2-propanyl)-methacrylsäureester

### a) Synthese

Eine Lösung von 97 g (0,2mol) 1,3-Bis(4-benzoyl-3-hydroxyphenoxy)-2-propanol und 103 g (1,2mol) Methacyrlsäure in 1200 ml Toluol wurde nach Zusatz von 6 g Trifluormethansulfonsäure, 0,2 g Hydrochinon und 0,2 g Hydrochinonmonomethylether 3 Stunden lang auf eine Temperatur von 105 bis 110^{o}C erhitzt. Nach dem Erkalten auf Raumtemperatur wurde das Reaktionsgemisch mit 31 Wasser gewaschen. Anschließend wurde das Toluol bei einem Druck von 20mbar aus dem Reaktionsgemisch abdestilliert. Der verbleibende ölige Rückstand wurde in heißem Ethanol gelöst. Nach dem Erkalten dieser Lösung auf Raumtemperatur erhielt man 86g (78% der Theorie) (1,3-Bis(4-benzoyl-3-hydroxy)-2-propanyl)-methacrylsäureester in Form farbloser Kristalle mit einem Schmelzpunkt von 138 bis 139^{o}C und einer Reinheit von 99% (HPLC). Das UV-Spektrum ergab zwei Absorptionsmaxima bei Wellenlängen von 287nm (Extinktion 0,517; Extinktionskoeffizient 28570) und 324nm (Extinktion 0,333; Extinktionskoeffizient 18400).

### b) Färbung

Ein Färbebad bestehend aus 1500 Teilen Wasser, 0,6 Teilen einer Farbstoffmischung aus
a) einer Gelbmischung aus
   0,08 Teilen Disperse Yellow 42,
   0,08 Teilen Disperse Yellow 86,
   0,08 Teilen Disperse Yellow 108,
b) einer Rotmischung aus
   0,035 Teilen Disperse Red 91,
   0,035 Teilen Disperse Red 92,
   0,035 Teilen Disperse Red 279,
c) einer Blaumischung aus
   0,091 Teilen Disperse Blue 77,
   0,091 Teilen Disperse Blue 56,
   0,091 Teilen Disperse Blue 27,
2 Teilen ®Dispersogen A (Dispergiermittel der Hoechst AG, Frankfurt, West Germany) und 1,5 Teilen der Verbindung aus a) wird mit Essigsäure auf einen pH-Wert von 4,5 - 5 eingestellt.

Beginnend bei 60^{o}C werden 100 Teile eines Polyestergarns in diesem Färbebad in einem HT-Färbeapparat gefärbt. Innerhalb von 30 Minuten wird die Temperatur auf 135^{o}C erhöht und weitere 90 Minuten bei 135^{o}C gefärbt.

Es wird eine hellgraue Färbung erhalten, die bei einer Belichtung im Xenontest unter den Bedingungen gemäß DIN 75202 deutlich bessere Lichtechtheiten erreicht als eine Färbung ohne Zusatz der Verbindung aus a).

### Beispiel 2

### (1,3-Bis(4-benzoyl-3-hydroxyphenoxy)-2-propanyl)-essigsäurereste

### a) Synthese

Eine Lösung von 484 g (1 Mol) 1,3-Bis(4-benzoyl-2-hydroxyphenoxy)-2-propanol und 360,3 g (6 Mol) Essigsäure in 2,6 l Toluol wurde nach Zusatz von 6 g Trifluormethansulfonsäure und 5 g Methansulfonsäure 5 Stunden lang am Wasserabscheider zum Rückfluß erhitzt, bis durch Abscheidung der berechneten Menge Wasser - 18 g - die Umsetzung erfolgt ist. Nach dem Erkalten auf Raumtemperatur wurde das Reaktionsgemisch mit 5 l Wasser gewaschen. Anschließend wurde das Toluol bei einem Druck von ca. 20 mbar aus dem Reaktionsgemisch abdestilliert. Der verbleibende Rückstand wurde zur Aufreinigung in einem Gemisch, bestehend aus Aceton/Ethanol, heiß gelöst.

Nach dem Erkalten dieser Lösung auf 15^{o}C erhielt man 421 g (80% der Theorie) (1,3-Bis(4-benzoyl-2-hydroxyphenoxy)-2-propanol)essigsäureester in Form feiner farbloser Kristalle und einer Reinheit von 99% (HPLG). UV-, IR- und NMR-Spektren entsprechen der genannten Verbindung.

### b) Färbung

100 Teile eines Polyestervelours werden in einem Färbebad analog Beispiel 1 b), das aber 1,5 Teile der Verbindung aus Beispiel 2a und eine Farbstoffmischung aus 0,43 Teilen Disperse Yellow 51, 0,46 Teilen einer Rotmischung wie in Beispiel 1b) und 0,17 Teilen Disperse Blue 77 enthält, gefärbt.

Man erhält eine altrosa Färbung, die bei einer Belichtung unter den Bedingungen nach DIN 75202 deutlich bessere Lichtechtheit erreicht, als eine Färbung ohne Zusatz der Verbindung des Beispiels 2a).

### Beispiel 3

### (1,3-Bis(4-benzoyl-3-hydroxyphenoxy)-2-propanyl)-benzoesäureester.

### a) Synthese

Eine Lösung von 200 g (0,41 Mol) 1,3-Bis(4-benzoyl-3-hydroxyphenoxy)-2-propanyl und 80 g Pyridin wurde bei einer Temperatur von 100 bis 105^{o}C innerhalb von 3 Stunden mit 141 g (1 Mol) Benzoylchlorid versetzt. Aus der klaren Lösung fiel ein feiner Niederschlag von Pyridinhydrochlorid aus. Die Reaktion wurde noch für 3 Stunden bei 100-105^{o}C vervollständigt. Nach dem Erkalten auf Raumtemperatur wurde das Reaktionsgemisch mit 2 l Wasser gewaschen. Anschließend wurde das Toluol bei einem Druck von ca. 20 mbar aus dem Reaktionsgemisch abdestilliert. Der verbleibende Rückstand wurde durch Umkristallisation gereinigt.

Das so erhaltene Produkt besitzt einen Schmelzpunkt von 168-169^{o}C und eine Reinheit von 99% (HPLG). UV-, IR und NMR-Spektren entsprechen der genannten Verbindung.

### b) Färbung

100 Teile eines Polyestergewirkes werden in einem Färbebad analog Beispiel 1b), das aber 2 Teile der Verbindung aus Beispiel 3a) und eine Farbstoffmischung aus 0,46 Teilen Disperse Yellow 42, 0,17 Teile einer Rotmischung wie in Beispiel 1b) und 0,17 Teile einer Blaumischung wie in Beispiel 1b) enthält, gefärbt.

Man erhält eine beige Färbung, die, belichtet nach DIN 75202, eine deutlich bessere Lichtechtheit erreicht, als eine vegleichbare Färbung ohne Zusatz der Verbindung des Beispiels 3a).

## Patentansprüche

1. Verbindung der allgemeinen Formel I 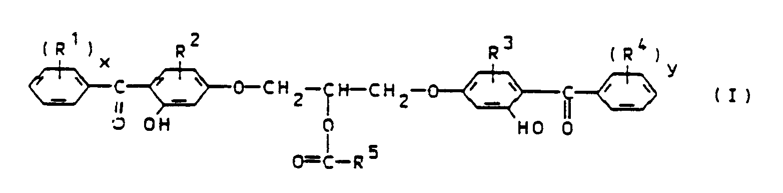 worin
R¹ und R⁴ unabhängig voneinander (C₁-C₆)-Alkyl, Cyan, Fluor, Chlor, Brom, Trifluormethyl oder Wasserstoff;
R² und R³ unabhängig voneinander (C₁-C₆)-Alkyl oder Wasserstoff;
x und y unabhängig voneinander 0, 1 oder 2 bedeuten und R⁵ für (C₁-C₁₂)-Alkyl; (C₃-C₈)-Cycloalkyl; durch (C₁-C₆)-Alkoxy, (C₂-C₇)-Alhoxycarbonyl, Amino, (C₁-C₆)-Alkylamino, Di-(C₂-C₆)-Alkylamino, Hydroxy, Fluor, Chlor, Brom, Trifluormethyl oder Nitro substituiertes (C₁-C₁₂)-Alkyl oder (C₃-C₈)-Cycloalkyl; Phenyl oder durch (C₁-C₆)-Alkyl, (C₁-C₆)-Alkoxy, (C₂-C₇)-Alkoxycarbonyl, Amino, (C₁-C₆)-Alkylamino, Di-(C₁-C₆)-Alkylamino, Hydroxy, Cyan, Fluor, Chlor, Brom, Trifluormethyl oder Nitro substituiertes Phenyl steht.

2. Verbindung der allgemeinen Formel I gemäß Anspruch 1, dadurch gekennzeichnet, daß R¹ und R⁴, unabhängig voneinander Wasserstoff, Chlor oder Trifluormethyl, (C₁-C₃)-Alkyl, Cyan, R² und R³ unabhängig voneinander Wasserstoff oder (C₁-C₃)-Alkyl,
x und y unabhängig voneinander 0 oder 1 und
R⁵ (C₁-C₄)-Alkyl; Cyclopentyl; Cyclohexyl, durch (C₁-C₄)-Alkoxy, (C₂-C₅)-Alkoxycarbonyl, Amino, (C₁-C₄)-Alkylamino, Di-(C₁-C₄)-Alkylamino, Hydroxy, Fluor, Chlor, Brom, Trifluormethyl oder Nitro substituiertes (C₁-C₄)-Alkyl, Cyclopentyl oder Cyclohexyl; Phenyl oder durch (C₁-C₄)-Alkyl, (C₁-C₄)-Alkoxy, (C₂-C₅)-Alkoxycarbonyl, Amino, (C₁-C₄)-Alkylamino, Di-(C₁-C₄)-Alkylamino, Hydroxy, Cyan, Fluor, Chlor, Brom, Trifluormethyl oder Nitro substituiertes Phenyl bedeuten.

3. Verbindung der allgemeinen Formel I gemäß Anspruch 1 und/oder 2, dadurch gekennzeichnet, daß
R¹, R², R³ und R⁴ Wasserstoff,
x und y O und
R⁵ Methyl oder Phenyl
bedeuten.

4. Verfahren zur Herstellung einer Verbindung der allgemeinen Formel I des Anspruchs 1, dadurch gekennzeichnet, daß man eine Verbindung der allgemeinen Formel II 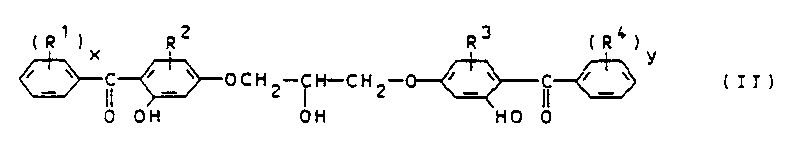 worin R¹, R², R³, R⁴, x und y wie oben definiert sind, mit einer Verbindung der allgemeinen Formel III 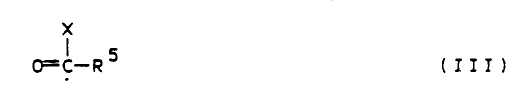 worin x für Hydroxy, Halogen, (C₁-C₄)-Alkoxy oder -OCOR⁵ oder R⁵ wie in Anspruch 1 definiert ist, umsetzt.

5. Verwendung einer Verbindung der allgemeinen Formel I 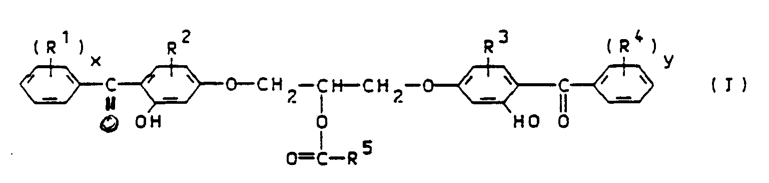 worin R¹ und R⁴ unabhängig voneinander (C₁-C₆)-Alkyl, Cyan, Fluor, Chlor, Brom, Trifluormethyl oder Wasser stoff;
R² oder R³ unabhängig voneinander (C₁-C₆)-Alkyl oder Wasserstoff;
x und y unabhängig voneinander 0, 1 oder 2 bedeuten und
R⁵ für (C₁-C₁₂)-Alkyl; (C₃-C₈)-Cycloalkyl; durch (C₁-C₆)-Alkoxy, (C₂-C₇)-Alkoxycarbonyl, Amino, (C₁-C₆)-Alkylamino, Di-(C₁-C₆)-Alkylamino, Hydroxy, Cyan, Fluor, Chlor, Brom, Trifluormethyl oder Nitro substituiertes (C₁-C₁₂)-Alkyl oder (C₃-C₈)-Cycloalkyl; Phenyl oder durch (C₁-C₆)-Alkyl, (C₁-C₆)-Alkoxy, (C₂-C₇)-Alkoxycarbonyl, Amino, (C₁-C₆)-Alkylamino, Di-(C₁-C₆)-Alkylamino, Hydroxy, Cyan, Fluor, Chlor, Brom, Trifluormethyl oder Nitro substituiertes Phenyl steht oder eine Gruppe der allgemeinen Formel A 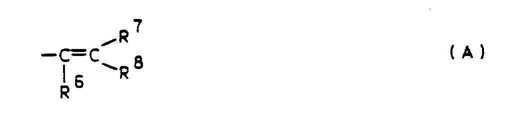 bedeutet, worin
R⁶ Wasserstoff, Phenyl, (C₁-C₁₂)-Alkyl oder Cyan und
R⁷ und R⁸ unabhängig voneinander Wasserstoff, (C₁-C₈)-Alkyl, Phenyl oder durch (C₁-C₄)-Alkyl oder (C₁-C₄)-Alkoxy substituiertes Phenyl bedeuten oder gemeinsam einen Alkylenrest mit 4 oder 5 Kohlenstoffatomen bilden, zur Verbesserung der Lichtechtheit von Polyesterfärbungen.

6. Verfahren zum Färben von textilem Polyestermaterial mit Dispersionsfarbstoffen, dadurch gekennzeichnet, daß das Färbebad zur Verbesserung der Lichtechtheit der Färbung mindestens eine Verbindung der allgemeinen Formel I 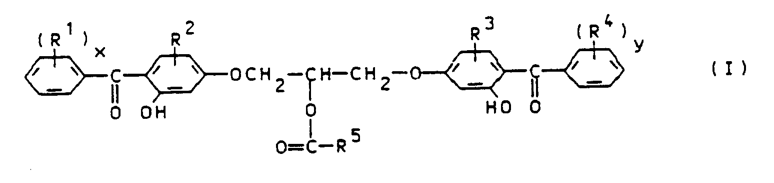 worin R¹ und R⁴ unabhängig voneinander (C₁-C₆)-Alkyl, Cyan, Fluor, Chlor, Brom, Trifluormethyl oder Wasserstoff;
R² und R³ unabhängig voneinander (C₁-C₆)-Alkyl oder Wasserstoff;
x und y unabhängig voneinander 0, 1 oder 2
bedeuten und
R⁵ für (C₁-C₁₂)-Alkyl; (C₃-C₈)-Cycloalkyl; durch (C₁-C₆)-Alkoxy, (C₂-C₇)-Alkoxycarbonyl, Amino, (C₁-C₆)-Alkylamino, Di-(C₁-C₆)-Alkylamino, Hydroxy, Cyan, Fluor, Chlor, Brom, Trifluormethyl oder Nitro substituiertes (C₁-C₁₂)-Alkyl oder (C₃-C₈)-Cycloalkyl; Phenyl oder durch (C₁-C₆)-Alkyl, (C₁-C₆)-Alkoxy, (C₂-C₇)-Alkoxycarbonyl, Amino, (C₁-C₆)-Alkylamino, Di-(C₁-C₆)-Alkylamino, Hydroxy, Cyan, Fluor, Chlor, Brom, Trifluormethyl oder Nitro substituiertes Phenyl steht oder eine Gruppe der allgemeinen Formel A 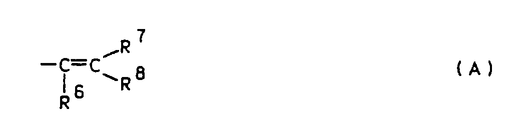 bedeutet, worin
R⁶ Wasserstoff, Phenyl, (C₁-C₁₂)-Alkyl oder Cyan und R⁷ und R⁸ unabhängig voneinander Wasserstoff, (C₁-C₈)-Alkyl, Phenyl oder durch (C₁-C₄)-Alkyl oder (C₁-C₄)-Alkoxy substituiertes Phenyl bedeuten oder gemeinsam einen Alkylenrest mit 4 oder 5 Kohlenstoffatomen bilden, enthält.

7. Verfahren gemäß Anspruch 6, dadurch gekennzeichnet, daß das Färbebad die Verbindung der allgemeinen Formel I in Mengen von 0,1 bis 10, besonders bevorzugt 0,3 bis 5 Gewichtsprozent, bezogen auf das zu färbende Textilgut, enthält.

8. Farbstoffpräparation, dadurch gekennzeichnet, daß sie mindestens eine Verbindung der allgemeinen Formel I 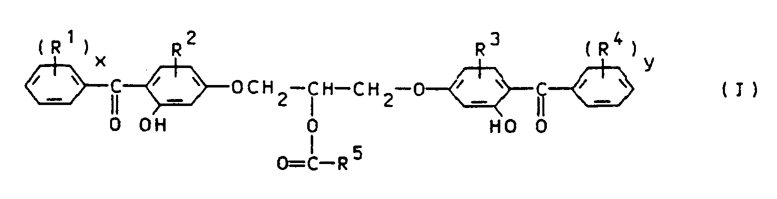 worin R¹ und R⁴ unabhängig voneinander (C₁-C₆)-Alkyl, Cyan, Fluor, Chlor, Brom, Trifluormethyl oder Wasserstoff;
R² und R³ unabhängig voneinander (C₁-C₆)-Alkyl oder Wasserstoff;
x und y unabhängig voneinander 0, 1 oder 2
bedeuten und
R⁵ für (C₁-C₁₂)-Alkyl; (C₃-C₈)-Cycloalkyl; durch (C₁-C₆)-Alkoxy, (C₂-C₇)-Alkoxycarbonyl, Amino, (C₁-C₆)-Alkylamino, Di-(C₁-C₆)-Alkylamino, Hydroxy, Cyan, Fluor, Chlor, Brom, Trifluormethyl oder Nitro substituiertes (C₁-C₁₂)-Alkyl oder (C₃-C₈)-Cycloalkyl; Phenyl oder durch (C₁-C₆)-Alkyl, (C₁-C₆)-Alkoxy, (C₂-C₇)-Alkoxycarbonyl, Amino, (C₁-C₆)-Alkylamino, Di-(C₁-C₆)-Alkylamino, Hydroxy, Cyan, Fluor, Chlor, Brom, Trifluormethyl oder Nitro substituiertes Phenyl steht oder eine Gruppe der allgemeinen Formel A 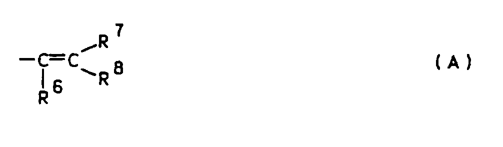 bedeutet, worin
R⁶ Wasserstoff, Phenyl, (C₁-C₁₂)-Alkyl oder Cyan und
R⁷ und R⁸ unabhängig voneinander Wasserstoff, (C₁-C₈)-Alkyl, Phenyl oder durch (C₁-C₄)-Alkyl oder (C₁-C₄)-Alkoxy substituiertes Phenyl bedeuten oder gemeinsam einen Alkylenrest mit 4 oder 5 Kohlenstoffatomen bilden, enthält.

9. Farbstoffpräparation gemäß Anspruch 8, dadurch gekennzeichnet, daß sie die Verbindungen der allgemeinen Formel I in Mengen von 1 bis 50 Gewichtsprozent, besonders bevorzugt 1 bis 30 Gewichtsprozent, enthält.

## Claims

1. Compound of the general formula I 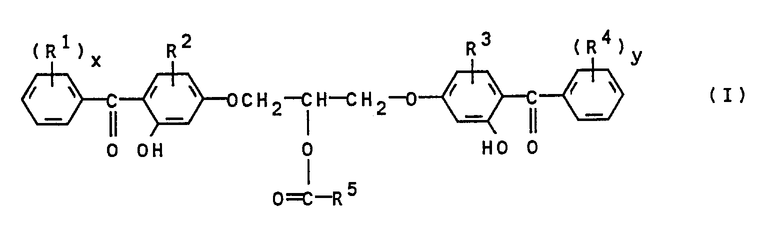 in which R¹ and R⁴, independently of one another, are (C₁-C₆)-alkyl, cyano, fluorine, chlorine, bromine, trifluoromethyl or hydrogen;
R² and R³, independently of one another, are (C₁-C₆)-alkyl or hydrogen;
x and y, independently of one another, are 0, 1 or 2 and R⁵ represents (C₁-C₁₂)-alkyl; (C₃-C₈)-cycloalkyl; (C₁-C₁₂)-alkyl or (C₃-C₈)-cycloalkyl, each of which is substituted by (C₁-C₆)-alkoxy, (C₂-C₇)-alkoxycarbonyl, amino, (C₁-C₆)-alkylamino, di-(C₂-C₆)-alkylamino, hydroxyl, fluorine, chlorine, bromine, trifluoromethyl or nitro; phenyl or phenyl substituted by (C₁-C₆)-alkyl, (C₁-C₆)-alkoxy, (C₂-C₇)-alkoxycarbonyl, amino, (C₁-C₆)-alkylamino, di-(C₁-C₆)-alkylamino, hydroxyl, cyano, fluorine, chlorine, bromine, trifluoromethyl or nitro.

2. Compound of the general formula I according to claim 1, characterized in that R¹ and R⁴, independently of one another, are hydrogen, chlorine or trifluoromethyl, (C₁-C₃)-alkyl, cyano, R² and R³, independently of one another, are hydrogen or (C₁-C₃)-alkyl,
x and y, independently of one another, are 0 or 1 and R⁵ is (C₁-C₄)-alkyl; cyclopentyl; cyclohexyl, (C₁-C₄)-alkyl, cyclopentyl or cyclohexyl, each of which is substituted by (C₁-C₄)-alkoxy, (C₂-C₅)-alkoxycarbonyl, amino, (C₁-C₄)-alkylamino, di-(C₁-C₄)-alkylamino, hydroxyl, fluorine, chlorine, bromine, trifluoromethyl or nitro; phenyl or phenyl substituted by (C₁-C₄)-alkyl, (C₁-C₄)-alkoxy, (C₂-C₅)-alkoxycarbonyl, amino, (C₁-C₄)-alkylamino, di-(C₁-C₄)-alkylamino, hydroxyl, cyano, fluorine, chlorine, bromine, trifluoromethyl or nitro.

3. Compound of the general formula I according to claim 1 and/or 2, characterized in that
R¹, R², R³ and R⁴ are hydrogen
x and y are 0 and
R⁵ is methyl or phenyl.

4. Process for the preparation of a compound of the general formula I of claim 1, characterized in that a compound of the general formula II 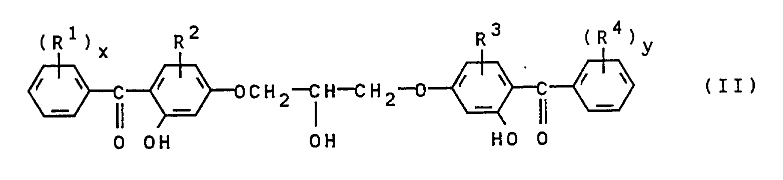 in which R¹, R², R³, R⁴, x and y are as defined above, is reacted with a compound of the general formula III 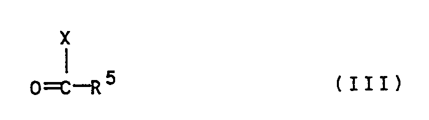 in which X represents hydroxyl, halogen, (C₁-C₄)-alkoxy or -OCOR⁵ or R⁵ is as defined as in claim 1.

5. Use of a compound of the general formula I 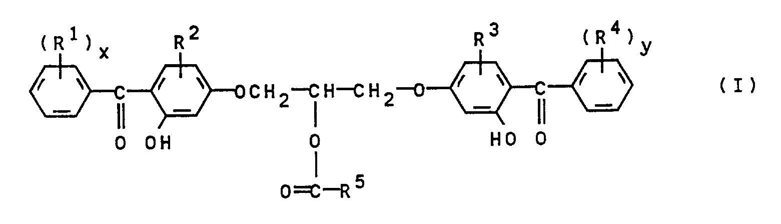 in which R¹ and R⁴, independently of one another, are (C₁-C₆)-alkyl, cyano, fluorine, chlorine, bromine, trifluoromethyl or hydrogen;
R² and R³, independently of one another, are (C₁-C₆)-alkyl or hydrogen,
x and y, independently of one another, are 0, 1 or 2 and R⁵ represents (C₁-C₁₂)-alkyl; (C₃-C₈)-cycloalkyl; (C₁-C₁₂)-alkyl or (C₃-C₈)-cycloalkyl, each of which is substituted by (C₁-C₆)-alkoxy, (C₂-C₇)-alkoxycarbonyl, amino, (C₁-C₆)-alkylamino, di-(C₁-C₆)-alkylamino, hydroxyl, cyano, fluorine, chlorine, bromine, trifluoromethyl or nitro; phenyl or phenyl substituted by (C₁-C₆)-alkyl, (C₁-C₆)-alkoxy, (C₂-C₇)-alkoxycarbonyl, amino, (C₁-C₆)-alkylamino, di-(C₁-C₆)-alkylamino, hydroxyl, cyano, fluorine, chlorine, bromine, trifluoromethyl or nitro, or a group of the general formula A 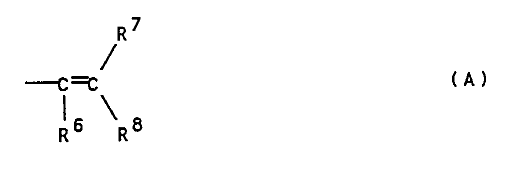 in which
R⁶ is hydrogen, phenyl, (C₁-C₁₂)-alkyl or cyano and
R⁷ and R⁸, independently of one another, are hydrogen, (C₁-C₈)-alkyl, phenyl or phenyl substituted by (C₁-C₄)-alkyl or (C₁-C₄)-alkoxy or together form an alkylene radical having 4 or 5 carbon atoms, for improving the light fastness of polyester dyeings.

6. Process for the dyeing of textile polyester material using disperse dyes, characterized in that the dye bath contains, for improving the light fastness of the dyeing, at least one compound of the general formula I 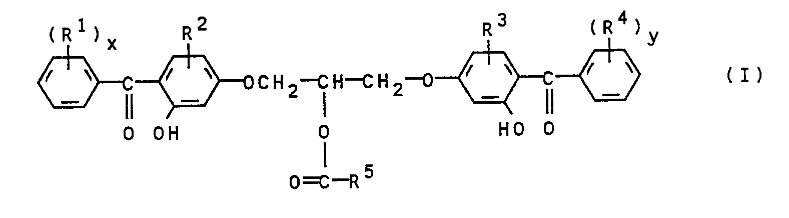 in which R¹ and R⁴, independently of one another, are (C₁-C₆)-alkyl, cyano, fluorine, chlorine, bromine, trifluoromethyl or hydrogen;
R² and R³, independently of one another, are (C₁-C₆)-alkyl or hydrogen;
x and y, independently of one another, are 0, 1 or 2 and
R⁵ represents (C₁-C₁₂)-alkyl; (C₃-C₈)-cycloalkyl; (C₁-C₁₂)-alkyl or (C₃-C₈)-cycloalkyl, each of which is substituted by (C₁-C₆)-alkoxy, (C₂-C₇)-alkoxycarbonyl, amino, (C₁-C₆)-alkylamino, di-(C₁-C₆)-alkylamino, hydroxyl, cyano, fluorine, chlorine, bromine, trifluoromethyl or nitro; phenyl or phenyl substituted by (C₁-C₆)-alkyl, (C₁-C₆)-alkoxy, (C₂-C₇)-alkoxycarbonyl, amino, (C₁-C₆)-alkylamino, di-(C₁-C₆)-alkylamino, hydroxyl, cyano, fluorine, chlorine, bromine, trifluoromethyl or nitro, or a group of the general formula A 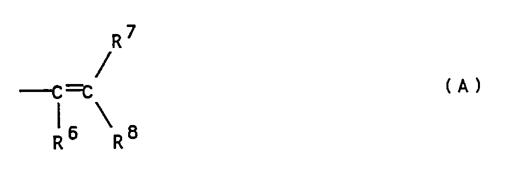 in which
R⁶ is hydrogen, phenyl, (C₁-C₁₂)-alkyl or cyano and
R⁷ and R⁸, independently of one another, are hydrogen, (C₁-C₈)-alkyl, phenyl or phenyl substituted by (C₁-C₄)-alkyl or (C₁-C₄)-alkoxy or together form an alkylene radical having 4 or 5 carbon atoms.

7. Process according to claim 6, characterized in that the dye bath contains the compound of the general formula I in amounts of 0.1 to 10, particularly preferably 0.3 to 5, percent by weight, relative to the textile material to be dyed.

8. Dye preparation, characterized in that it contains at least one compound of the general formula I 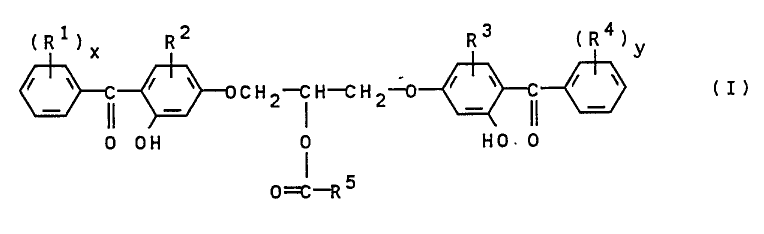 in which R¹ and R⁴, independently of one another, are (C₁-C₆)-alkyl, cyano, fluorine, chlorine, bromine, trifluoromethyl or hydrogen;
R² and R³, independently of one another, are (C₁-C₆)-alkyl or hydrogen,
x and y, independently of one another, are 0, 1 or 2 and
R⁵ represents (C₁-C₁₂)-alkyl; (C₃-C₈)-cycloalkyl; (C₁-C₁₂)-alkyl or (C₃-C₈)-cycloalkyl, each of which is substituted by (C₁-C₆)-alkoxy, (C₂-C₇)-alkoxycarbonyl, amino, (C₁-C₆)-alkylamino, di-(C₁-C₆)-alkylamino, hydroxyl, cyano, fluorine, chlorine, bromine, trifluoromethyl or nitro; phenyl or phenyl substituted by (C₁-C₆)-alkyl, (C₁-C₆)-alkoxy, (C₂-C₇)-alkoxycarbonyl, amino, (C₁-C₆)-alkylamino, di-(C₁-C₆)-alkylamino, hydroxyl, cyano, fluorine, chlorine, bromine, trifluoromethyl or nitro, or a group of the general formula A 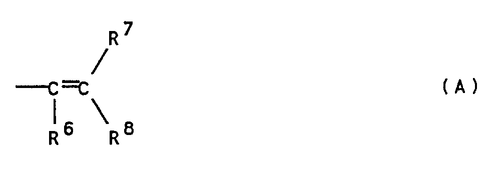 in which
R⁶ is hydrogen, phenyl, (C₁-C₁₂)-alkyl or cyano and
R⁷ and R⁸, independently of one another, are hydrogen, (C₁-C₈)-alkyl, phenyl or phenyl substituted by (C₁-C₄)-alkyl or (C₁-C₄)-alkoxy or together form an alkylene radical having 4 or 5 carbon atoms.

9. Dye preparation according to claim 8, characterized in that it contains the compounds of the general formula I in amounts of 1 to 50 percent by weight, particularly preferably 1 to 30 percent by weight.

## Revendications

1. Composés de formule générale I 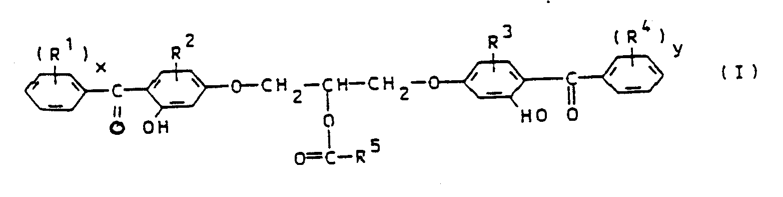 dans laquelle
R¹ et R⁴ indépendamment l'un de l'autre représentent les alkyle en C₁-C₆, cyan, fluor, chlore, brome, trifluorométhyle ou hydrogène ;
R² et R³ indépendamment l'un de l'autre représentent un alkyle en C₁-C₆ ou l'hydrogène.
x et y indépendamment l'un de l'autre, représente 0, 1 ou 2, et
R⁵ représente un alkyle en C₁-C₁₂ ; un cycloalkyle en C₃-C₈; un cycloalkyle en C₃-C₈ ou un alkyle en C₁-C₁₂ substitué par des alcoxy en C₁-C₆, alcoxycarbonyle en C₂-C₇, amino, alkylamino en C₁-C₆, dialkyl(C₁-C₆)amino, hydroxy, fluor, chlore, brome, trifluorométhyle ou nitro ; un phényle ou un phényle substitué par des alkyle en C₁-C₆, alcoxy en C₁-C₆, alcoxycarbonyle en C₂-C₇, amino, alkylamino en C₁-C₆, dialkyl(C₁-C₆)amino, hydroxy, cyan, fluor, chlore, brome, trifluorométhyle ou nitro.

2. Composés selon la formule générale I selon la revendication 1, caractérisés en ce que
R¹ et R⁴ indépendamment l'un de l'autre représentent les hydrogène, chlore, alkyle en C₁-C₃, cyan, ou trifluorométhyle,
R² et R³ indépendamment l'un de l'autre représentent l'hydrogène ou un alkyle en C₁-C₃,
x et y indépendamment l'un de l'autre représentent 0 ou 1, et
R⁵ signifie un alkyle en C₁-C₄ ; un cyclopentyle, un cyclohexyle ; un cyclohexyle, un cyclopentyle ou un alkyle en C₁-C₄ substitué par des alcoxy en C₁-C₄, alcoxycarbonyle en C₂-C₅, amino, alkylamino en C₁-C₄, dialkyl(C₁-C₄)amino, hydroxy, fluor, chlore, brome, trifluorométhyle ou nitro ; un phényl ou un phényl substitué par des alkyle en C₁-C₄, alcoxy en C₁-C₄, alcoxycarbonyle en C₂-C₅, amino, alkylamino en C₁-C₄, dialkyl(C₁-C₄)amino_{,} hydroxy, cyan, fluor, chlore, brome, trifluorométhyle ou nitro.

3. Composés de formule générale I selon la revendication 1 et/ou 2, caractérisés en ce que
R¹, R², R³ et R⁴ représentent l'hydrogène,
x et y représentent 0, et
R⁵ représente le méthyle ou le phényle.

4. Procédé pour la préparation de composés de formule générale I de la revendication 1, caractérisé en ce qu'on fait réagir un composé de formule générale II 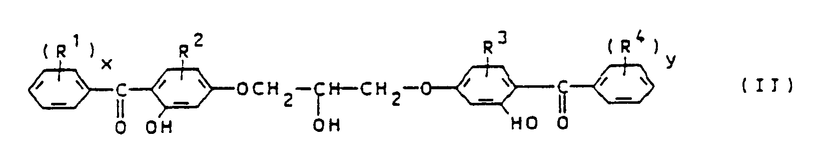 dans laquelle R¹, R², R³, R⁴, x et y sont définis comme ci-dessus, avec un composé de formule générale III 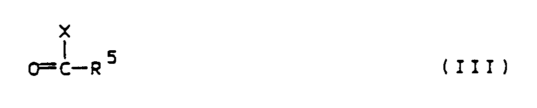 dans laquelle X représente les hydroxy, halogène, alcoxy en C₁-C₄ ou -OCOR⁵, ou R⁵ est défini comme dans la revendication 1.

5. Utilisation d'un composé de formule générale I 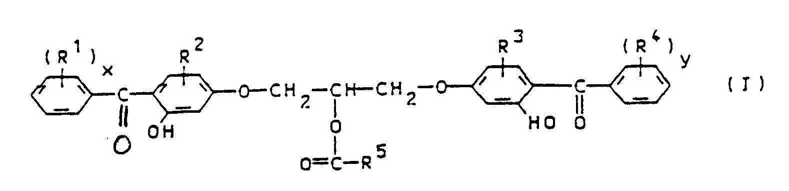 dans laquelle
R¹ et R⁴ indépendamment l'un de l'autre représentent les alkyle en C₁-C₆, cyan, fluor, chlore, brome, trifluorométhyle ou hydrogène ;
R² et R³ indépendamment l'un de l'autre représentent un alkyle en C₁-C₆ ou l'hydrogène.
x et y indépendamment l'un de l'autre, représentent 0, 1 ou 2, et
R⁵ représente un alkyle en C₁-C₁₂ ; un cycloalkyle en C₃-C₈; un cycloalkyle en C₃-C₈ ou un alkyle en C₁-C₁₂ substitué par des alcoxy en C₁-C₆, alcoxycarbonyle en C₂-C₇, amino, alkylamino en C₁-C₆, dialkyl(C₁-C₆)amino, hydroxy, cyan, fluor, chlore, brome, trifluorométhyle ou nitro ; un phényle ou un phényle substitué par un alkyle en C₁-C₆, un alcoxyle en C₁-C₆, un alcoxycarbonyle en C₂-C₇, un amino, un alkylamino en C₁-C₆, un dialkyl(C₁-C₆)amino, un hydroxy, un cyan, le fluor, le chlore, le brome, le trifluorométhyle ou un nitro, ou un groupe de formule générale A 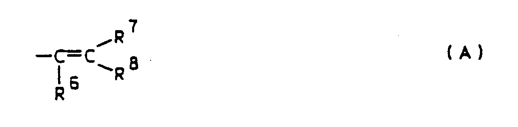
dans laquelle
R⁶ représente les hydrogène, phényle, alkyle en C₁-C₁₂ ou cyan, et
R⁷ et R⁸ indépendamment l'un de l'autre représentent les hydrogène, alkyle en C₁-C₈, phényle ou phényle substitué par des alkyle en C₁-C₄ ou alcoxy en C₁-C₄, ou ensemble forment un radical alkylène comportant de 4 ou 5 atomes de carbone, pour l'amélioration de la solidité à la lumière des teintures de polyesters.

6. Procédé pour la teinture de matières textiles polyesters avec des colorants dispersés caractérisés en ce que le bain de teinture pour l'amélioration de la solidité à la lumière de la teinture contient au moins un composé de formule générale I dans laquelle : 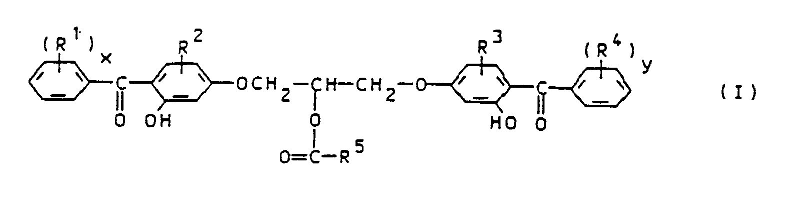
R¹ et R⁴ indépendamment l'un de l'autre représentent les alkyle en C₁-C₆, cyan, fluor, chlore, brome, trifluorométhyle ou hydrogène ;
R² et R³ indépendamment l'un de l'autre représentent un alkyle en C₁-C₆ ou l'hydrogène.
x et y indépendamment l'un de l'autre représentent 0, 1 ou 2, et
R⁵ représente un alkyle en C₁-C₁₂ ; un cycloalkyle en C₃-C₈; un cycloalkyle en C₃-C₈ ou un alkyle en C₁-C₁₂ substitué par des alcoxy en C₁-C₆, alcoxycarbonyle en C₂-C₇, amino, alkylamino en C₁-C₆, dialkyl(C₁-C₆)amino, hydroxy, cyan, fluor, chlore, brome, trifluorométhyle ou nitro ; un phényle ou un phényle substitué par des alkyle en C₁-C₆, alcoxy en C₁-C₆, alcoxycarbonyle en C₂-C₇, amino, alkylamino en C₁-C₆, dialkyl(C₁-C₆)amino, hydroxy, cyan, fluor, chlore, brome, trifluorométhyle ou nitro, ou un groupe de formule générale A 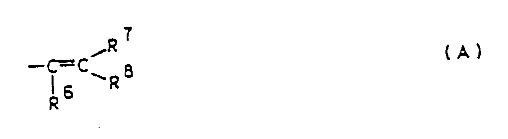
dans laquelle
R⁶ représente les hydrogène, phényle, alkyle en C₁-C₁₂ ou cyan, et
R⁷ et R⁸ indépendamment l'un de l'autre représentent les hydrogène, alkyle en C₁-C₈, phényle ou phényle substitué par des alkyle en C₁-C₄ ou alcoxy en C₁-C₄, ou ensemble forment un radical alkylène comportant 4 ou 5 atomes de carbone.

7. Procédé selon la revendication 6, caractérisé en ce que le bain de teinture contient le composé de formule générale I, en quantité de 0,1 à 10, de manière particulièrement préférée de 0,3 à 5% en poids, par rapport au produit textile à teindre.

8. Préparation de colorants caractérisée en ce qu'elle contient au moins un composé de formule générale I 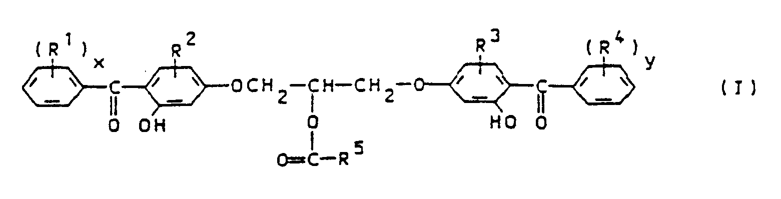 dans laquelle
R¹ et R⁴ indépendamment l'un de l'autre représentent les alkyle en C₁-C₆, cyan, fluor, chlore, brome, trifluorométhyle ou hydrogène ;
R² et R³ indépendamment l'un de l'autre représentent un alkyle en C₁-C₆ ou l'hydrogène.
x et y indépendamment l'un de l'autre représentent 0, 1 ou 2 et
R⁵ représente un alkyle en C₁-C₁₂ ; un cycloalkyle en C₃-C₈; un cycloalkyle en C₃-C₈ ou un alkyle en C₁-C₁₂ substitué par des alcoxy en C₁-C₆, alcoxycarbonyle en C₂-C₇, amino, alkylamino en C₁-C₆, dialkyl(C₁-C₆)amino, hydroxy, cyan, fluor, chlore, brome, trifluorométhyle ou nitro ; un phényle ou un phényle substitué par des alkyle en C₁-C₆, alcoxy en C₁-C₆, alcoxycarbonyle en C₂-C₇, amino, alkylamino en C₁-C₆, dialkyl(C₁-C₆)amino hydroxy, cyan, fluor, chlore, brome, trifluorométhyle ou nitro, ou groupe de formule générale A 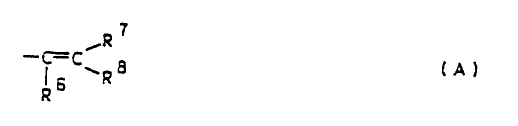
dans laquelle
R⁶ représente les hydrogène, phényle, alkyle en C₁-C₁₂ ou cyan, et
R⁷ et R⁸ indépendamment l'un de l'autre représentent les hydrogène, alkyle en C₁-C₈, phényle ou phényle substitué par des alkyle en C₁-C₄ ou alcoxy en C₁-C₄, ou ensemble forment un radical alkylène comportant 4 ou 5 atomes de carbone.

9. Préparation de colorant selon la revendication 8, caractérisée en ce qu'elle contient le composé de formule générale I en quantité de 1 à 50% en poids, de manière particulièrement préférée de 1 à 30% en poids.
